(19) Europäisches Patentamt
European Patent Office
Office européen des brevets

(11) Publication number: 0 012 621 B2

(12) # NEW EUROPEAN PATENT SPECIFICATION

(45) Date of publication of the new patent specification: 22.03.89

(21) Application number: 79302914.1

(22) Date of filing: 17.12.79

(51) Int. Cl.⁴: A 61 K 31/19, A 61 K 31/195, A 61 K 31/40 // (A61K31/19, 31:165),(A61K31/195, 31:165),(A61K31/40, 31:165)

(54) **Analgesic composition comprising acetaminophen as a potentiating agent.**

(30) Priority: 18.12.78 US 970513
18.12.78 US 970685
18.12.78 US 970520
18.12.78 US 970519
18.12.78 US 970512
18.12.78 US 970511
18.12.78 US 970510

(43) Date of publication of application: 25.06.80 Bulletin 80/13

(45) Publication of the grant of the patent: 08.06.83 Bulletin 83/23

(45) Mention of the opposition decision: 22.03.89 Bulletin 89/12

(84) Designated Contracting States: AT BE CH DE FR GB IT NL SE

(56) References cited:
DE-A-1 941 583
GB-A-1 293 768
GB-A-1 302 169
US-A-3 068 147
US-A-3 439 094
ZA-A-732 294

CHEMICAL ABSTRACTS, Vol.82, No. 19, May 12,1975, page 38, abstract 118998c. Columbus, Ohio, USA, P. WARRICK et al. "Interactions in rats between the nonsteriodal antiinflammatory drugs, aspirin and fenoprofen".
J. Int. Med. Res., Vol. 6, p. 375 (1968)
J. Int. Med. Res., Vol. 3, p. 139 (1975)

(73) Proprietor: McNeilab, Inc., Camp Hill Road, Fort Washington Pennsylvania 19034 (US)

(72) Inventor: Gardocki, Joseph Francis, 72 Meadow Lane, Doylestown, PA, 18901 (US)

(74) Representative: Jones, Alan John et al, CARPMAELS & RANSFORD 43 Bloomsbury Square, London, WC1A 2RA (GB)

(56) References cited: (continuation)
Physician's Deck Reference, p. 899, 900 (1983)
Br. J. Anaest.,Vol. 50, p. 931-935 (1978)
Schweiz. Med. W. schr., Bd. 105, S. 652 (1975)
Arch. Int. Pharmacodyn, Vol. 155, p. 365 (1965)
Arch. Int. Pharmacodyn, Vol. 235, p. 116 (1978)
Brit. J. Pharmac., Vol. 32, p. 295-310 (1968)
Brit. Med. J. p. 165 (1972)
A Folha Médica, Vol. 71, p. 341 (1975)

The file contains technical information submitted after the application was filed and not included in this specification

EP 0 012 621 B2

## Description

This invention is directed to improved methods and compositions for producing analgesia.

Background of the Invention

One of the long-existing primary goals of medicine is the relief of pain. Relief is sought most generally by the administration of analgesic drugs which produce a state of decreased awareness of the sensation and increase of the pain threshold.

Almost all potent analgesics evoke reactions other than the relief of pain. Some of the reactions are gastrointestinal disturbances, nausea, constipation and vomiting. Among the more serious of the side reactions and one frequently found in analgesic drugs is respiratory depression. Thus, in the use of analgesics in man, considerations other than the primary effect (analgesia), must be made and drugs for pain relief are sought which have maximum analgesic effect accompanied by minimum side reactions. It is difficult to satisfy these requirements with a single chemical entity since generally a potent analgesic has accompanying serious side reactions while a drug with little or no side effects are generally less effective as an analgesic.

Thus, there is a continuing search for a combination of two or more drugs whereby the total quantity of drug can be reduced and which can be employed in such proportions as to produce maximum analgesic effect with little or no side effects. When one or both of the components of a combination is known to possess pain relieving properties, but these properties are increased many-fold, the net effect of the combination is commonly referred to as "potentiation".

Acetaminophen (p-acetaminophenol) is recognized as an analgesic agent with useful and safe antinociceptive properties. However, in certain instances, high doses must be employed to effectively reduce pain. The following drugs, designated as A through F, respectively, have analgesic properties,

A) 2-[(2,6-Dichlorophenyl)amino]benzeneacetic acid is known by the generic name of "diclofenac" for use as an anti-inflammatory agent; it also has some analgesic properties. It is represented by the formula

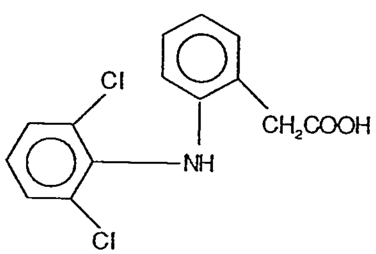

B) (Z)-5-Fluoro-2-methyl-1-{[4-methylsulfinyl)-phenyl]-methylene}-1H-indene-3-acetic acid is known by the generic name of "sulindac" for use as an antiinflammatory agent; it also has analgesic properties. It is represented by the formula

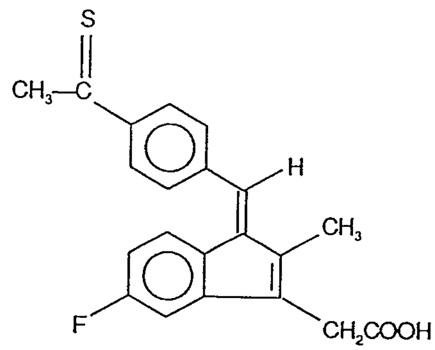

C) 3-(4-Biphenylcarbonyl)-propionic acid is known by the generic name of "fenbufen" for use as an anti-inflammatory agent; it also has some analgesic properties. It is represented by the formula

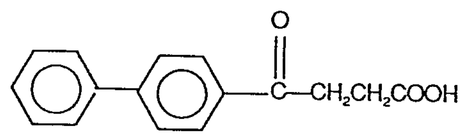

D) 6-Methoxy-α-methyl-2-naphthaleneacetic acid is known by the generic name of "naproxen" for use as anti-inflammatory, analgesic or antipyretic agent. It is represented by the formula

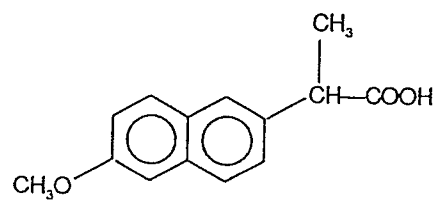

E) m-Benzoylhydratropic acid is known by the generic name of "ketoprofen" for use as an anti-inflammatory agent; it also has some analgesic properties. It is represented by the formula

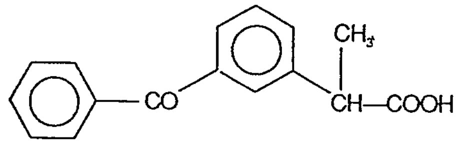

F) 4-(1,3-Dihydro-1-oxo-2H-isoindol-2-yl)-α-methylbenzeneacetic acid is known by the generic name of "indoprofen" for use as an anti-inflammatory and as an analgesic agent. It is represented by the formula

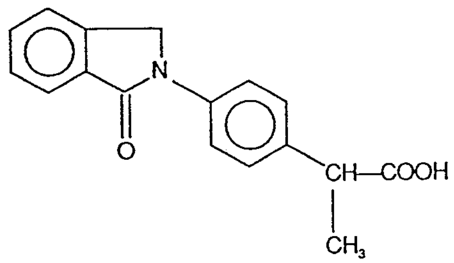

However, the effect of the combination of acetaminophen and any one of drugs A through F on the analgesic properties was not known before our work.

Dürrigl et al., J. Int. Med. Res., Vol. 3, No. 3, page 139 (1975), Pinheiro et al., A Folha Médica, Vol. 71, page 341 (1975) and Wagenhäuser et al., Schweiz. med. Wschr., Vol. 105, page 652 (1975) describe comparative trials of diclofenac and indomethacin for efficacy against arthritis (rheumatoid and osteoarthritis) and tolerability. Acetaminophen was available to the patients as a rescue analgesic.

Statement of the Invention

The present invention concerns an improved method of producing analgesia made possible by the discovery that a potentiation of analgesic or antinociceptive properties of any one of drugs A through F or their salts is produced by administration with acetaminophen in specific proportions. The use of the combination in the suppression of pain is unexpectedly much greater than that which would result from simply the additive effect of the components.

Description of the Invention

The novel and unexpected superior analgesic properties may be achieved by the simultaneous oral administration of (1) acetaminophen and (2) one of said compounds A through F, or therapeutically acceptable salts thereof. The therapeutically acceptable salts are those obtained from appropriate organic or inorganic bases. Preferred salts include these of potassium and the like.

The efficacy of the novel combination in producing antinociceptive properties is particularly seen in acetylcholine bromide abdominal constriction assay of Collier, et al. [(Brit. J. Pharmacol. Chemotherap. 32, 295-310 (1968)] sometimes referred to as the "mouse writhing test". In the test, mice are dosed with test drug combinations and thereafter injected interperitoneally with acetylcholine bromide and the abdominal constriction responses or block of abdominal constriction responses are observed and compared with control operations.

More specifically, in an operation carried out substantially as described by Collier and coworkers, non-fasted albino mice weighing 18-24 grams were dosed with combinations of one test drug in selected fixed doses together with a second test drug at variable doses for each fixed dose. These were compared with animals dosed with (a) the first test drug at the same doses used in combination but employing saline instead of the second drug, (b) the second test drug at the doses used in combination but employing saline instead of the first drug, and (c) a saline control containing no drug. Each of the drugs, when separately employed, were found to be inactive in the acetylcholine bromide abdominal constriction test at the test dose level.

In carrying out the tests, the test compositions and the control compositions both with and without drugs were administered orally to the test mice. After about 30 minutes the mice were injected intraperitoneally with acetylcholine bromide and the abdominal constriction responses compared (corrected for saline responses as necessary). The results are expressed as percent block.

When the percent block of abdominal construction observed with the fixed dose was 5 per cent or more, the percent block observed with each of the combination dosage levels was corrected with respect to the percent block observed with a fixed dose drug using Abbott's formula for natural mortality.

Likewise, when the reponse to acetylcholine bromide in the saline control group was 95 per cent or less, the response observed with each of the variable dosage levels used in the $ED_{50}$ determination was similarly corrected using Abbott's formula for natural mortality. $ED_{50}$ and 95 per cent confidence limits were calculated according to Finney's probit analysis procedure. (Finney, D.J. 1964, Probit Analysis, Second Edition, University Press, Cambridge.) All $ED_{50}$ for a given comparison were calculated using a common slope following a test for parallelism. All paired dose response curves were found to be parallel.

Compound A

Employing the above-described procedures, potentiation of the analgesic properties of a 2-[(2,6-dichlorophenyl)amino]-benzeneacetic acid compound (hereinafter sometimes referred to as "diclofenac compound") by acetaminophen may be demonstrated. The property is illustrated employing the sodium salt of diclofenac but is to be understood that it is not limited thereto.

Non-fasted male albino mice weighing 18-24 grams were dosed initially per os (p.o.) with diclofenac sodium at various doses and with saline as seen in Tables 1a and 1b and fifteen minutes later with acetaminophen at 25 mg/kg or at 50 mg/kg. All dose levels of test drug and saline control were given as and are expressed as per os. Twenty mice were employed for each dosage level. As controls, a similar number of mice were dosed with (a) acetaminophen (25 mg/kg) plus saline (5 ml/kg) or acetaminophen (50 mg/kg) plus saline (5 ml/kg) and (b) double dose of saline (5 ml/kg). Diclofenac sodium was administered as an aqueous solution. Acetaminophen was administered as an aqueous suspension.

Thirty minutes after administration of diclofenac sodium (and 15 minutes after administration of acetaminophen), the mice were injected intraperitoneally (i.p.) with 5.5 mg/kg of acetylcholine bromide and observed for the presence or absence of the abdominal constriction response. $ED_{50}$ values were determined from the observed values applying where pertinent corrections previously discussed. The results are seen in Tables 1a and 1b.

(Where "mg/kg" or "ml/kg" is employed, "kg" is in reference to body weight.)

Table 1a

| Treatment | Diclofenac sodium dose (mg/kg) | Observation number responding / number injected | Percent block | Diclofenac $ED_{50}$ (95% C.L.***) (mg/kg) |
|---|---|---|---|---|
| Diclofenac Na plus saline, 5 ml/kg | 2.0 | 0/20 | 100 (100)* | 0.37 (0.23–0.61)+ |
| | 1.0 | 2/20 | 90 (88)* | 0.34 (0.21–0.57)++ |
| | 0.5 | 8/20 | 60 (53)* | |
| | 0.25 | 13/20 | 35 (24)* | |
| | 0.10 | 17/20 | 15 (0)* | |
| Diclofenac Na plus acetaminophen, 25 mg/kg | 1.0 | 1/20 | 95 (94)** | 0.19 (0.13–0.27)+ |
| | 0.5 | 3/20 | 85 (82)** | 0.18 (0.12–0.25)++ |
| | 0.25 | 7/20 | 65 (59)** | |
| | 0.10 | 11/20 | 45 (35)** | |
| | 0.05 | 17/20 | 15 (0)** | |
| Acetaminophen, 25 mg/kg plus saline, 5 ml/kg | — | 17/20 | 15 (0) | |
| Saline, 5 ml/kg plus saline, 5 ml/kg | — | 17/20 | 15 | |

* Percent block corrected for saline response
** Percent block corrected for acetaminophen plus saline response
*** Confidence limits
\+ Calculated as salt
\++ Calculated as acid

Table 1b

| Treatment | Diclofenac sodium dose (mg/kg) | Observation number responding / number injected | Percent block | Diclofenac $ED_{50}$ (95% C.L.***) (mg/kg) |
|---|---|---|---|---|
| Diclofenac Na plus saline, 5 ml/kg | 5.0 | 0/20 | 100 (100)* | 0.37 (0.26–0.54)+ |
| | 2.5 | 1/20 | 95 (95)* | 0.34 (0.24–0.50)++ |
| | 1.0 | 4/20 | 80 (79)* | |
| | 0.5 | 7/20 | 65 (63)* | |
| | 0.25 | 13/20 | 35 (32)* | |
| | 0.10 | 17/20 | 15 (11)* | |
| Diclofenac Na plus acetaminophen, 50 mg/kg | 0.5 | 1/20 | 95 (92)** | 0.09 (0.04–0.19)+ |
| | 0.25 | 4/20 | 80 (67)** | 0.08 (0.04–0.18)++ |
| | 0.10 | 6/20 | 70 (50)** | |
| | 0.05 | 8/20 | 60 (33)** | |
| | 0.025 | 16/20 | 20 (0)** | |
| Acetaminophen, 50 mg/kg plus saline, 5 ml/kg | — | 12/20 | 40 (37) | |
| Saline, 5 ml/kg plus saline, 5 ml/kg | — | 19/20 | 5 | |

* Percent block corrected for saline response
** Percent block corrected for acetaminophen plus saline response
*** Confidence limits
\+ Calculated as salt
\++ Calculated as acid

Another experiment was carried out in a manner described in the foregoing example except that acetaminophen was employed only at the 50 mg/kg level. The results are seen in Table I′.

(Where "mg/kg" or "ml/kg" is employed, "kg" is in reference to body weight).

Table 1′

| Treatment | Diclofenac sodium dose (mg/kg) | Observation number responding / number injected | Percent block | Diclofenac $ED_{50}$ (95% C.L.***) (mg/kg) |
|---|---|---|---|---|
| Diclofenac Na plus saline, 5 ml/kg | 2.5 | 1/20 | 95 (94)* | 0.49 (0.29–0.84)+ |
| | 1.0 | 7/20 | 65 (61)* | 0.46 (0.27–0.78)++ |
| | 0.5 | 9/20 | 55 (50)* | |
| | 0.25 | 12/20 | 40 (33)* | |
| | 0.10 | 17/20 | 15 (6)* | 0.15 (0.08–0.29)+ |
| Diclofenac Na plus acetaminophen, 50 mg/kg | 1.0 | 2/20 | 90 (87)** | 0.14 (0.07–0.27)++ |
| | 0.25 | 6/20 | 70 (60)** | |
| | 0.10 | 9/20 | 55 (40)** | |
| | 0.05 | 11/20 | 45 (27)** | |
| | 0.025 | 13/20 | 35 (13)** | |
| Acetaminophen, 50 mg/kg plus saline, 5 ml/kg | — | 15/20 | 27 (17) | |
| Saline, 5 ml/kg plus saline, 5 ml/kg | — | 18/20 | 10 | |

* Percent block corrected for saline response
** Percent block corrected for acetaminophen plus saline response
*** Confidence limits
\+ Calculated as salt
\+ Calculated as acid

The foregoing results illustrated the potentiation of the analgesic or antinociceptive properties of a diclofenac compound by acetaminophen.

The present process, namely, a method of producing analgesia, comprises orally administering to subjects, i.e., human and other warm-blooded animals suffering from pain, one of compounds A through F and acetaminophen in amounts sufficient to have an antinociceptive effect. By the administration of the amounts of the agents as hereinafter set forth, an antinociceptive interaction between the drugs is achieved which is wholly unexpected from the known properties of the components. One method of administration is by use of compositions in unit dosage form which provides a convenient simultaneous administration method.

From the foregoing test results on mice and the known dosages ranges of the components as applied to man when employed alone, it is determined that in the case of Compound A, from 0.08 to 0.47 mg/kg of diclofenac compound may be employed together with from 1.8 to 7.5 mg/kg of acetaminophen; preferably from 0.17 to 0.33 mg/kg of diclofenac compound with from 2.1 to 7.1 mg/kg of acetaminophen. These amounts when expressed as doses suitable for man are in the range of from 5 to 28 milligrams of diclofenac compound and from 110 to 450 milligrams of acetaminophen; preferably from 10 to 20 milligrams of diclofenac compound and from 125 to 425 milligrams of acetaminophen.

The outstanding properties may be effectively, utilized by use of the novel pharmaceutical compositions of the present invention. To prepare the pharmaceutical compositions of this invention, one of Compounds A through F as primary action agent and acetaminophen as a potentiating agent, are intimately admixed with a pharmaceutically acceptable carrier suitable for oral administration. In preparing the compositions in oral dosage form, any of the usual pharmaceutical media may be employed, including liquid carriers such as water, glycols, oils, alcohols and the like for oral liquid preparations such as suspensions, elixirs and solutions; and solid carriers such as starches, sugars, kaolin, calcium stearate, ethyl celluose, etc. including materials which function as lubricants, binders, disintegrating agents and the like for powders, capsules and tablets. Because of their ease in administration, tablets and capsules represent the most advantageous oral dosage form, these compositions employ solid pharmaceutical carriers such as the aforementioned starches, sugars, kaolin and the like, and generally with a lubricant such as calcium stearate. It is especially advantageous to formulate the aforementioned pharmaceutical compositions in dosage unit form for ease of administration and uniformity of dosage. The term "dosage unit form" as used in the specification and claims herein refers to physically discrete units suitable as unitary dosages, each unit containing a predetermined quantity of active ingredient calculated

to produce the desired therapeutic effect in association with the required pharmaceutical carrier. Examples of such dosage unit forms are tablets, capsules, pills, powder packets, wafers, teaspoonsful, tablespoonsful and the like, and segregated multiples thereof. In the case of Compound A, a dosage unit generally will contain from 5 to 28 milligrams of a 2-[(2,6-dichlorophenyl)-amino]benzeneacetic acid compound as primary active ingredient together with from 110 to 450 milligrams of acetaminophen. The preferred dosage unit (when using Compound A) is from 10 to 20 milligrams of a 2-[(2,6-dichlorophenyl)amino]-benzeneacetic acid compound together with from 125 to 425 milligrams of acetaminophen.

Compound B

Employing the above-described procedures, potentiation of the analgesic properties of a (Z)-5-fluoro-2-methyl-1-{[4-(methylsulfinyl)phenyl]methylene}-1-H-indene-3-acetic acid compound (hereinafter sometimes referred to as "sulindac compound") by acetaminophen may be demonstrated. The property is illustrated by employing sulindac (acid form) but is to be understood that it is not limited thereto.

Non-fasted male albino mice weighing 18–24 grams were dosed initially per os (p.o.) with sulindac at various doses and with saline as seen in Tables 2a and 2b and fifteen minutes later with acetaminophen at 25 mg/kg or at 50 mg/kg. All dose levels of test drug and saline control were given as and are expressed as per os. Twenty mice were employed for each dosage level. As controls, a similar number of mice were dosed with (a) acetaminophen (25 mg/kg) plus saline (5 ml/kg) or acetaminophen (50 mg/kg) plus saline (5 ml/kg) and (b) double dose of saline (5 ml/kg). Sulindac was administered as an aqueous suspension. Acetaminophen was administered as an aqueous suspension.

Thirty minutes after administration of sulindac (and 15 minutes after administration of acetaminophen, the mice were injected intraperitoneally (i.p.) with 5.5 mg/kg of acetylcholine bromide and observed for the presence or absence of the abdominal constriction response. $ED_{50}$ values were determined from the observed values applying where pertinent corrections previously discussed. The results are seen in Tables 2a and 2b.

(Where "mg/kg" or "ml/kg" is employed, "kg" is in reference to body weight).

Table 2a

Antinociceptive activity of sulindac and acetaminophen in the mouse body constriction assay.
Time 30 minutes post sulindac, 15 min. post acetaminophen.

| Treatment | Sulindac dose mg/kg p.o. | Observation number responding / number injected | % Block | Corrected % block | Sulindac $ED_{50}$ (95% C.L.***) mg/kg p.o. |
|---|---|---|---|---|---|
| Sulindac plus saline, 5 ml/kg p.o. | 25 | 2/20 | 90 | 88* | 12.3 (9.4–16.1) |
| | 15 | 6/20 | 70 | 65* | |
| | 10 | 11/20 | 45 | 35* | |
| | 7.5 | 13/20 | 35 | 24* | |
| | 5 | 17/20 | 15 | 0* | |
| Sulindac plus acetaminophen, 25 mg/kg p.o. | 15 | 1/20 | 95 | 94** | 4.6 (3.4–6.3) |
| | 10 | 3/20 | 85 | 81** | |
| | 5 | 8/20 | 60 | 50** | |
| | 2.5 | 12/20 | 40 | 25** | |
| | 1.0 | 17/20 | 15 | 0** | |
| Acetaminophen, 25 mg/kg p.o. plus saline, 5 ml/kg | — | 16/20 | 20 | 6 | |
| Saline, 5 ml/kg p.o. plus saline, 5 ml/kg p.o. | — | 17/20 | 15 | | |

* % Block in this column corrected for saline response
** % Block in this column corrected for acetaminophen plus saline response
*** Confidence limits

Table 2b

Antinociceptive activity of sulindac + acetaminophen in the mouse body constrictive assay. Time 30 minutes post sulindac, 15 min. post acetaminophen.

| Treatment | Sulindac dose mg/kg p.o. | Observation number responding / number injected | % Block | Corrected % block | $ED_{50}$ (95% C.L.***) mg/kg p.o. |
|---|---|---|---|---|---|
| Sulindac plus saline, 5 ml/kg p.o. | 25 | 4/20 | 80 | 79* | 12.5 (8.8–17.8) |
| | 15 | 8/20 | 60 | 58* | |
| | 10 | 12/20 | 40 | 37* | |
| | 5 | 16/20 | 20 | 16* | |
| | 2.5 | 17/20 | 15 | 11* | |
| Sulindac plus acetaminophen, 50 mg/kg p.o. | 20 | 0/20 | 100 | 100** | 3.3 (1.9–5.7) |
| | 10 | 3/20 | 85 | 79** | |
| | 5 | 6/20 | 70 | 57** | |
| | 2.5 | 9/20 | 55 | 36** | |
| | 1.0 | 12/20 | 40 | 14** | |
| Acetaminophen, 50 mg/kg p.o. plus saline, 5 ml/kg p.o. | — | 14/20 | 30 | 26 | |
| Saline, 5 ml/kg p.o. plus saline, 5 ml/kg p.o. | — | 19/20 | 5 | — | |

* % Block in this column corrected for saline response

** % Block in this column corrected for acetaminophen plus saline response

*** Confidence limits

From the foregoing test results on mice and the known dosage ranges of the components as applied to man when employed alone, it is determined that generally from 0.1 to 1.7 mg/kg of sulindac compound may be employed together with from 1.0 to 7.5 mg/kg of acetaminophen; preferably from 0.17 to 1.5 mg/kg of sulindac compound with from 1.7 to 7.1 mg/kg of acetaminophen. These amounts when expressed as doses suitable for man are in the range of from 6 to 100 milligrams of sulindac compound and from 65 to 450 milligrams of acetaminophen; preferably from 10 to 90 milligrams of sulindac compound and from 100 to 425 milligrams of acetaminophen.

Examples of dosage unit forms are tablets, capsules, pills, powder packets, wafers, teaspoonsful, tablespoonsful and the like, and segregated multiples thereof. A dosage unit generally will contain from 6 to 100 milligrams of a sulindac compound as primary active ingredient together with from 65 to 450 milligrams of acetaminophen. The preferred dosage unit is from 10 to 90 milligrams of a sulindac compound together with from 100 to 425 milligrams of acetaminophen.

Compound C

Employing the above-described procedures, potentiation of the analgesic properties of a 3-(4-biphenylylcarbonyl)-propionic acid compound (hereinafter sometimes referred to as "fenbufen compound") by acetaminophen may be demonstrated. The property is illustrated employing fenbufen (acid form) but it is to be understood that it is not limited thereto.

Non-fasted male albino mice weighing 18–24 grams were dosed initially per os (p.o.) with fenbufen at various doses and with saline as seen in Tables 3a and 3b and fifteen minutes later with acetaminophen at 25 mg/kg or at 50 mg/kg. All dose levels of test drug and saline control were given as and are expressed as per os. Twenty mice were employed for each dosage level. As controls, a similar number of mice were dosed with (a) acetaminophen (25 mg/kg) plus saline (5 ml/kg) or acetaminophen (50 mg/kg) plus saline (5 ml/kg) and (b) double dose of saline (5 ml/kg). Fenbufen was administered as an aqueous suspension. Acetaminophen was administered as an aqueous suspension.

Thirty minutes after administration of fenbufen (and 15 minutes after administration of acetaminophen), the mice were injected intraperitoneally (i.p.) with 5.5 mg/kg of acetylcholine bromide and observed for the presence or absence of the abdominal constriction response. $ED_{50}$ values were determined from the observed values applying where pertinent corrections previously discussed. The results are seen in Tables 3a and 3b.

(Where "mg/kg" or "ml/kg" is employed, "kg" is in reference to body weight.)

Table 3a

| Treatment | Fenbufen dose (mg/kg) | Observation number responding / number injected | Percent block | Fenbufen $ED_{50}$ (95% C.L.***) (mg/kg) |
|---|---|---|---|---|
| Fenbufen plus saline, 5 ml/kg | 75 | 2/20 | 90 (88)* | 35.3 (26.0–48.0) |
| | 50 | 6/20 | 70 (65)* | |
| | 35 | 9/20 | 55 (47)* | |
| | 25 | 13/20 | 35 (24)* | |
| | 10 | 17/20 | 15 (0)* | |
| Fenbufen plus acetaminophen, 25 mg/kg | 50 | 1/20 | 95 (94)** | 16.8 (12.5–22.6) |
| | 25 | 5/20 | 75 (69)** | |
| | 15 | 8/20 | 60 (50)** | |
| | 10 | 14/20 | 30 (13)** | |
| | 5 | 16/20 | 20 (10)** | |
| Acetaminophen, 25 mg/kg + saline, 5 ml/kg | — | 16/20 | 20 (6) | |
| Saline, 5 ml/kg plus saline, 5 ml/kg | — | 17/20 | 15 (—) | |

* Percent block corrected for saline response
** Percent block corrected for acetaminophen plus saline response
*** Confidence limits

Table 3b

| Treatment | Fenbufen dose (mg/kg) | Observation number responding / number injected | Percent block | Fenbufen $ED_{50}$ (95% C.L.***) (mg/kg) |
|---|---|---|---|---|
| Fenbufen plus saline, 5 ml/kg | 75 | 3/20 | 85 (83)* | 34.3 (22.0–53.3) |
| | 50 | 8/20 | 60 (56)* | |
| | 25 | 11/20 | 45 (39)* | |
| | 15 | 13/20 | 35 (28)* | |
| | 10 | 15/20 | 25 (6)* | |
| Fenbufen plus acetaminophen, 50 mg/kg | 50 | 2/20 | 90 (88)** | 11.1 (6.7–18.4) |
| | 25 | 5/20 | 75 (69)** | |
| | 10 | 9/20 | 55 (44)** | |
| | 5 | 11/20 | 45 (31)** | |
| | 2.5 | 14/20 | 30 (13)** | |
| Acetaminophen, 50 mg/kg plus saline, 5 ml/kg | — | 16/20 | 20 (11) | |
| Saline, 5 ml/kg plus saline, 5 ml/kg | — | 18/20 | 10 (—) | |

* Percent block corrected for saline response
** Percent block corrected for acetaminophen plus saline response
*** Confidence limits

From the foregoing test results on mice and the known dosage ranges of the components as applied to man when employed alone, it is determined that generally from 2.2 to 7.2 mg/kg of fenbufen compound may be employed together with from 1.9 to 7.5 mg/kg of acetaminophen; preferably from 2.5 to 6.7 mg/kg of fenbufen compound with from 2.1 to 7.1 mg/kg of acetaminophen. These amounts when expressed as doses suitable for man are in the range of from 129 to 430 milligrams of fenbufen compound and from 113 to 450 milligrams of acetaminophen; preferably from 150 to 400 milligrams of fenbufen compound and from 125 to 425 milligrams of acetaminophen.

Examples of dosage unit forms are tablets, capsules, pills, powder packets, wafers, teaspoonsful, tablespoonsful and the like, and segregated multiples thereof. A dosage unit generally will contain from 129 to 430 mg of a 3-(4-bi-

phenylylcarbonyl)propionic acid compound as primary active ingredient together with from 113 to 450 mg of acetaminophen. The preferred dosage unit is from 150 to 400 mg of a 3-(4-biphenylylcarbonyl)propionic acid compound together with from 125 to 425 mg of acetaminophen.

Compound D

Employing the above-described procedures, potentiation of the analgesic properties of a 6-methoxy-α-methyl-2-naphthaleneacetic acid compound (hereinafter sometimes referred to as "naproxen compound") by acetaminophen may be demonstrated. The property is illustrated employing naproxen (acid form) but it is to be understood that it is not limited thereto.

Non-fasted male albino mice weighing 18–24 grams were dosed initially per os (p.o.) with naproxen at various doses and with saline as seen in Tables 4a and 4b and fifteen minutes later with acetaminophen at 25 mg/kg or at 50 mg/kg. All dose levels of test drug and saline control were given as and are expressed as per os. Twenty mice were employed for each dosage level. As controls, a similar number of mice were dosed with (a) acetaminophen (25 mg/kg) plus saline (10 ml/kg) or acetaminophen (50 mg/kg) plus saline (10 ml/kg) and (b) double dose of saline (10 ml/kg). Naproxen was administered as an aqueous suspension. Acetaminophen was administered as an aqueous suspension.

Thirty minutes after administration of naproxen (and 15 minutes after administration of acetaminophen), the mice were injected intraperitoneally (i.p.) with 5.5 mg/kg of acetylcholine bromide and observed for the presence or absence of the abdominal constriction response. $ED_{50}$ values were determined from the observed values applying where pertinent corrections previously discussed. The results are seen in Tables 4a and 4b.

(Where "mg/kg" or "ml/kg" is employed, "kg" is in reference to body weight.)

Table 4a

| Treatment | Naproxen dose (mg/kg) | Observation number responding / number injected | % Block | Corrected % block | Naproxen $ED_{50}$ (95% C.L.***) mg/kg po |
|---|---|---|---|---|---|
| Naproxen plus saline, 10 ml/kg | 20 | 4/20 | 80 | 75* | 7.99 (4.03–15.9) |
| | 10 | 8/20 | 60 | 50* | |
| | 5 | 9/20 | 55 | 44* | |
| | 3 | 13/20 | 35 | 19* | |
| Naproxen plus acetaminophen, 25 mg/kg | 20 | 3/20 | 85 | 80** | |
| | 10 | 6/20 | 70 | 60** | 7.52 (3.79–14.93) |
| | 5 | 12/20 | 40 | 20** | |
| | 3 | 11/20 | 45 | 27** | |
| Acetaminophen, 25 mg/kg plus saline, 10 ml/kg | — | 15/20 | 25 | 7 | |
| Saline, 10 ml/kg plus saline, 10 ml/kg | — | 16/20 | 20 | 0 | |

* Percent block corrected for saline response
** Percent block corrected for acetaminophen plus saline response
*** Confidence limits

Table 4b

| Treatment | Naproxen dose (mg/kg) | Observation number responding / number injected | % Block | Corrected** % block | Naproxen $ED_{50}$ (95% C.L.***) mg/kg po |
|---|---|---|---|---|---|
| Naproxen plus saline, 10 ml/kg | 20 | 2/20 | 90 | 86 | 8.91 (6.12–13.0) |
| | 10 | 5/20 | 75 | 64 | |
| | 8 | 10/20 | 50 | 29 | |
| | 5 | 12/20 | 40 | 14 | |
| | 2.5 | 15/20 | 25 | 0 | |
| Naproxen plus acetaminophen, 50 mg/kg | 10 | 1/20 | 95 | 93 | 3.10 (2.1–4.6) |
| | 5 | 2/20 | 90 | 87 | |
| | 4 | 8/20 | 60 | 43 | |
| | 2.5 | 10/20 | 50 | 29 | |
| | 1 | 13/20 | 35 | 7 | |

Table 4b (cont.)

| Treatment | Naproxen dose (mg/kg) | Observation number responding / number injected | % Block | Corrected** % block | Naproxen $ED_{50}$ (95% C.L.***) mg/kg po |
|---|---|---|---|---|---|
| Acetaminophen, 50 mg/kg plus saline, 10 ml/kg | — | 14/20 | 30 | 0 | |
| Saline, 10 ml/kg plus saline, 10 ml/kg | — | 14/20 | 30 | — | |

* Corrected for saline response
** Corrected for acetaminophen plus saline response
*** Confidence limits

From the foregoing test results on mice and the known dosage ranges of the components as applied to man when employed alone, it is determined that generally from 1.4 to 2.1 mg/kg of naproxen compound may be employed together with from 2.8 to 7.5 mg/kg of acetaminophen. These amounts when expressed as doses suitable for man are in the range of from 85 to 125 milligrams of naproxen compound and from 170 to 450 milligrams of acetaminophen.

Examples of dosage unit forms are tablets, capsules, pills, powder packets, wafers, teaspoonsful, tablespoonsful and the like and segregated multiples thereof. A dosage unit generally will contain from 85 to 125 milligrams of a 6-methoxy-α-2-methylnaphthaleneacetic acid compound as primary active ingredient together with from 170 to 450 milligrams of acetaminophen.

Compound E

Employing the above-described procedures, potentiation of the analgesic properties of a m-benzoylhydratropic acid compound (hereinafter sometimes referred to as "ketoprofen compound") by acetaminophen may be demonstrated. The property is illustrated employing ketoprofen (acid form) but it is to be understood that it is not limited thereto.

Non-fasted male albino mice weighing 18–24 grams were dosed initially per os (p.o.) with ketoprofen at various doses and with saline as seen in Tables 5a and 5b, and fifteen minutes later with acetaminophen at 25 mg/kg or at 50 mg/kg. All dose levels of test drug and saline controls were given as and are expressed as per os. Twenty mice were employed for each dosage level. As controls, a similar number of mice were dosed with (a) acetaminophen (25 mg/kg) plus saline (10 ml/kg) or acetaminophen (50 mg/kg) plus saline (10 ml/kg) and (b) double dose of saline (10 ml/kg). Ketoprofen was administered as a solution in aqueous sodium hydroxide. Acetaminophen was administered as an aqueous suspension. (Where "mg/kg" or "ml/kg" is employed, "kg" is in reference to body weight.)

Twenty minutes after administration of ketoprofen (and fifteen minutes after administration of acetaminophen), the mice were injected intraperitoneally (i.p.) with 5.5 mg/kg or acetylcholine bromide and observed for the presence or absence of the abdominal constriction response. $ED_{50}$ values were determined from the observed values applying were pertinent corrections previously discussed. The results are seen in Table 5a and 5b.

(Where "mg/kg" or "ml/kg" is employed, "kg" is in reference to body weight.)

Table 5a

| Treatment | Ketoprofen dose (mg/kg) | Observation number responding / number injected | Percent block | Ketoprofen $ED_{50}$ (95% CL***) (mg/kg) |
|---|---|---|---|---|
| Ketoprofen plus saline, 10 ml/kg | 5.0 | 0/20 | 100 (100)* | 0.47 (0.31–0.71) |
| | 1.0 | 3/20 | 85 (83)* | |
| | 0.5 | 10/20 | 50 (44)* | |
| | 0.25 | 13/20 | 35 (28)* | |
| | 0.10 | 17/20 | 15 (6)* | |
| Ketoprofen plus acetaminophen, 25 mg/kg | 1.0 | 3/20 | 85 (83)** | 0.31 (0.21–0.47) |
| | 0.5 | 5/20 | 75 (72)** | |
| | 0.25 | 12/20 | 40 (33)** | |
| | 0.10 | 15/20 | 25 (17)** | |

Table 5a (cont.)

| Treatment | Ketoprofen dose (mg/kg) | Observation number responding / number injected | Percent block | Ketoprofen $ED_{50}$ (95% CL***) (mg/kg) |
|---|---|---|---|---|
| Acetaminophen, 25 mg/kg plus saline, 10 ml/kg | — | 18/20 | 10 (0) | |
| Saline, 10 ml/kg plus saline, 10 ml/kg | — | 18/20 | 10 (0) | |

* Percent block corrected for saline response
** Percent block corrected for acetaminophen plus saline response
*** Confidence limits

Table 5b

| Treatment | Ketoprofen dose (mg/kg) | Observation number responding / number injected | Percent block | Ketoprofen $ED_{50}$ (95% C.L.***) (mg/kg) |
|---|---|---|---|---|
| Ketoprofen plus saline, 10 ml/kg | 1.0 | 1/20 | 95 (94)* | 0.46 (0.29–0.71) |
| | 0.75 | 5/20 | 75 (71)* | |
| | 0.50 | 11/20 | 45 (35)* | |
| | 0.25 | 15/20 | 25 (12)* | |
| Ketoprofen plus acetaminophen, 50 mg/kg | 1.0 | 0/20 | 100 (100)** | 0.19 (0.13–0.28) |
| | 0.5 | 2/20 | 90 (86)** | |
| | 0.25 | 6/20 | 70 (57)** | |
| | 0.10 | 11/20 | 45 (21)** | |
| | 0.05 | 12/20 | 40 (14)** | |
| Acetaminophen, 50 mg/kg plus saline, 10 ml/kg | — | 14/20 | 30 (18) | |
| Saline, 10 ml/kg plus saline, 10 ml/kg | — | 17/20 | 15 (—) | |

* Percent block corrected for saline response
** Percent block corrected for acetaminophen plus saline response
*** Confidence limits

From the foregoing test results on mice and the known dosage ranges of the components as applied to man when employed alone, it is determined that generally from 0.2 to 0.83 mg/kg of ketoprofen compound may be employed together with from 2.8 to 7.5 mg/kg of acetaminophen; preferably from 0.33 to 0.67 mm/kg of ketoprofen compound with from 3.3 to 7.1 mg/kg of acetaminophen. These amounts when expressed as doses suitable for man are in the range of from 12 to 50 milligrams of ketoprofen compound and from 170 to 450 milligrams of acetaminophen; preferably from 20 to 40 milligrams of ketoprofen compound and from 200 to 245 milligrams of acetaminophen.

Examples of dosage unit forms are tablets, capsules, pills, powder packets, wafers, teaspoonsful, tablespoonsful, and the like, and segregated multiples thereof. A dosage unit generally will contain from 12 to 50 mg of m-benzoylhydrotropic acid compound (ketoprofen compound) as primary active ingredient together with from 170 to 450 mg of acetaminophen. The preferred dosage unit is from 20 to 40 mg of a m-benzoylhydratropic acid compound together with from 200 to 425 mg of acetaminophen.

Compound F

Employing the above-described procedures, potentiation of the analgesic properties of a 4-(1,3-dihydro-1-oxo-2H-isoindol-2-yl)-α-methylbenzeneacetic acid compound (hereinafter sometimes referred to as "indoprofen compound") by acetaminophen may be demonstrated. The property is illustrated employing indoprofen (acid form) but it is to be understood that it is not limited thereto.

Non-fasted male albino mice weighing 18-24 grams were dosed initially per os (p.o.) with indoprofen at various doses and with saline as seen in Tables 6a and 6b and fifteen minutes later with acetaminophen at 25 mg/kg or at 50 mg/kg. All

dose levels of test drug and saline controls were given as and are expressed as per os. Twenty mice were employed for each dosage level. As controls, a similar number of mice were dosed with (a) acetaminophen (25 mg/kg) plus saline (5 ml/kg) or acetaminophen (50 mg/kg) plus saline (5 ml/kg) and (b) double dose of saline (5 ml/kg). Indoprofen was administered as an aqueous suspension. Acetaminophen was administered as an aqueous suspension.

Thirty minutes after administration of indoprofen (and 15 minutes after administration of acetaminophen), the mice were injected intraperitoneally (i.p.) with 5.5 mg/kg of acetylcholine bromide and observed for the presence or absence of the abdominal constriction response. $ED_{50}$ values were determined from the observed values applying where pertinent corrections previously discussed. The results are seen in Tables 6a and 6b.

(Where "mg/kg" or "ml/kg" is employed, "kg" is in reference to body weight.)

Table 6a

| Treatment | Indoprofen dose (mg/kg) | Observation number responding / number injected | Percent block | Indoprofen $ED_{50}$ (95% C.L.***) mg/kg |
|---|---|---|---|---|
| Indoprofen plus saline, 5 ml/kg | 5.0 | 3/20 | 85 (82)* | 1.73 (1.18–2.55) |
| | 2.5 | 6/20 | 70 (65)* | |
| | 1.5 | 8/20 | 60 (53)* | |
| | 1.0 | 13/20 | 35 (24)* | |
| | 0.5 | 16/20 | 20 (6)* | |
| Indoprofen plus acetaminophen, 25 mg/kg | 5.0 | 1/20 | 95 (93)** | 1.20 (0.72–2.01) |
| | 2.5 | 5/20 | 75 (67)** | |
| | 1.0 | 8/20 | 60 (47)** | |
| | 0.5 | 12/20 | 40 (20)** | |
| | 0.25 | 15/20 | 25 (0)** | |
| Acetaminophen, 25 mg/kg plus saline, 5 ml/kg | — | 15/20 | 25 (12) | |
| Saline, 5 ml/kg plus saline, 5 ml/kg | — | 17/20 | 15 (—) | |

* Percent block in this column corrected for saline response
** Percent block in this column corrected for acetaminophen plus saline response
*** Confidence limits

Table 6b

| Treatment | Indoprofen dose (mg/kg) | Observation number responding / number injected | Percent block | Indoprofen $ED_{50}$ (95% C.L.***) (mg/kg) |
|---|---|---|---|---|
| Indoprofen plus saline, 5 ml/kg | 5 | 0/20 | 100 (100)* | 1.58 (1.12–2.45) |
| | 3.5 | 2/20 | 90 (88)* | |
| | 2.5 | 8/20 | 60 (53)* | |
| | 1.0 | 12/20 | 40 (29)* | |
| | 0.5 | 16/20 | 20 (6)* | |
| Indoprofen plus acetaminophen, 50 mg/kg | 1.0 | 2/20 | 90 (87)** | 0.37 (0.23–0.57) |
| | 0.5 | 5/20 | 75 (67)** | |
| | 0.25 | 12/20 | 40 (20)** | |
| | 0.10 | 16/20 | 20 (0)** | |
| Acetaminophen, 50 mg/kg plus saline, 5 ml/kg | — | 15/20 | 25 (-12) | |
| Saline, 5 ml/kg plus saline, 5 ml/kg | — | 17/20 | 15 (-—) | |

* Percent block corrected for saline response
** Percent block corrected for acetaminophen plus saline response
*** Confidence limits

From the foregoing test results on mice and the known dosage ranges of the components as applied to man when employed alone, it is determined that generally from 0.17 to 2.5 mg/kg of indoprofen compound may be employed together with from 2.2 to 7.5 mg/kg of acetaminophen; preferably from 0.58 to 2.2 mg/kg of indoprofen compound with from 2.5 to 7.1 mg/kg of acetaminophen. These amounts when expressed as doses suitable for man are in the range of from 25 to 150 milligrams of indoprofen compound and from 135 to 450 milligrams of acetaminophen; preferably, from 35 to 130 milligrams of indoprofen compound and from 150 to 425 milligrams of acetaminophen.

Examples of dosage unit forms are tablets, capsules, pills, powder packets, wafers, teaspoonsful, tablespoonful, and the like, and segregated multiples thereof. A dosage unit generally will contain from 25 to 150 mg of an indoprofen compound as primary active ingredient together with from 150 to 450 mg of acetaminophen. The preferred dosage unit is from 35 to 130 mg of an indoprofen compound together with from 150 to 425 mg of acetaminophen.

The following examples are given to illustrate the novel compositions and are not to be construed as limiting the invention in spirit or in scope.

Compound A

Example 1

1000 hard gelatin capsules, each containing 15 milligrams of 2-[2,6-dichlorophenyl)amino]-benzeneacetic acid (diclofenac) as primary active ingredient and 280 milligrams of acetaminophen as potentiating agent are prepared from the following formulation:

| | Grams |
|---|---|
| Diclofenac | 15 |
| Acetaminophen | 280 |
| Starch | 250 |
| Lactose | 750 |
| Talc | 250 |
| Calcium stearate | 10 |

A uniform mixture of the ingredients is prepared by blending, and used to fill two-piece hard gelatin capsules. The capsules are suitable to be used for providing satisfactory analgesic effect upon adminstration to subjects suffering from pain.

Example 2

Gelatin capsules are prepared as described in Example 1 except that in the formulation, 15 grams of sodium 2-[2,6-dichlorphenyl)amino]benzeneacetate (diclofenac sodium) is employed as the primary active ingredient and 280 grams of acetaminophen is employed as the potentiating agent, thus providing capsules each containing 15 milligrams of diclofenac sodium and 280 milligrams of acetaminophen.

Example 3

1000 compressed tablets, each containing as the primary active ingredient 5 milligrams of diclofenac and 450 milligrams of acetaminophen as pontentiating agent are prepared from the following formulation:

| | Grams |
|---|---|
| Diclofenac | 5 |
| Acetaminophen | 450 |
| Starch | 750 |
| Dibasic calcium phosphate hydrous | 5000 |
| Calcium stearate | 2.5 |

The finely powdered ingredients are mixed well and granulated with 10 per cent starch paste. The granulation is dried and compressed into tablets.

Compound B

Example 4

1000 hard gelatin capsules, each containing 10 milligrams of sulindac as primary active ingredient and 425 milligrams of acetaminophen as potentiating agent are prepared from the following formulation:

| | Grams |
|---|---|
| Sulindac | 10 |
| Acetaminophen | 425 |
| Starch | 250 |
| Lactose | 750 |
| Talc | 250 |
| Calcium stearate | 10 |

A uniform mixture of the ingredients is prepared by blending, and used to fill two-piece hard gelatin capsules. The capsules are suitable to be used for providing satisfactory analgesic effect upon administration to subjects suffering from pain.

Example 5

1000 compressed tablets, each containing as the primary active ingredient 10 milligrams of sulindac and 425 milligrams of acetaminophen as potentiating agent are prepared from the following formulation:

| | Grams |
|---|---|
| Sulindac | 10 |
| Acetaminophen | 425 |
| Starch | 750 |
| Dibasic calcium phosphate hydrous | 5000 |
| Calcium stearate | 2.5 |

The finely powdered ingredients are mixed well and granulated with 10 per cent starch paste. The granulation is dried and compressed into tablets.

Compound C

Example 6

1000 hard gelatin capsules, each containing 275 milligrams of 3-(4-biphenylylcarbonyl)-propionic

acid (fenbufen) as primary active ingredient and 275 milligrams of acetaminophen as potentiating agent are prepared from the following formulation:

| | Grams |
|---|---|
| Fenbufen | 275 |
| Acetaminophen | 275 |
| Starch | 250 |
| Lactose | 750 |
| Talc | 250 |
| Calcium stearate | 10 |

A uniform mixture of the ingredients is prepared by blending, and used to fill two-piece hard gelatin capsules. The capsules are suitable to be used for providing satisfactory analgesic effect upon administration to subjects suffering from pain.

Example 7

1000 compressed tablets, each containing as the primary active ingredient 275 milligrams of fenbufen and 275 milligrams of acetaminophen as potentiating agent are prepared from the following formulation:

| | Grams |
|---|---|
| Fenbufen | 275 |
| Acetaminophen | 275 |
| Starch | 750 |
| Dibasic calcium phosphate hydrous | 5000 |
| Calcium stearate | 2.5 |

The finely powdered ingredients are mixed well and granulated with 10 per cent starch paste. The granulation is dried and compressed into tablets.

Compound D

Example 8

1000 hard gelatin capsules, each containing 105 milligrams of (+)-6-methoxy-α-methyl-2-naphthaleneacetic acid (naproxen) as primary active ingredient and 310 milligrams of acetaminophen as potentiating agent are prepared from the following formulation:

| | Grams |
|---|---|
| Naproxen | 105 |
| Acetaminophen | 310 |
| Starch | 250 |
| Lactose | 750 |
| Talc | 250 |
| Calcium stearate | 10 |

A uniform mixture of the ingredients is prepared by blending, and used to fill two-piece hard gelatin capsules. The capsules are suitable to be used for providing satisfactory analgesic effect upon administration to subjects suffering from pain.

Example 9

Gelatin capsules are prepared as described in Example 1 except that in the formulation, 115 grams of sodium 6-methoxy-α-methyl-2-naphtaleneacetate (naproxen sodium) is employed as the primary active ingredient and 350 grams of acetaminophen is employed as the potentiating agent, thus providing capsules each containing 115 milligrams of naproxen sodium and 350 milligrams of acetaminophen.

Example 10

1000 compressed tablets, each containing as the primary active ingredient 90 milligrams of naproxen and 425 milligrams of acetaminophen as pontentiating agent are prepared from the following formulation:

| | Grams |
|---|---|
| Naproxen | 90 |
| Acetaminophen | 425 |
| Starch | 750 |
| Dibasic calcium phosphate hydrous | 5000 |
| Calcium stearate | 2.5 |

The finely powdered ingredients are mixed well and granulated with 10 per cent starch paste. The granulation is dried and compressed into tablets.

Compound E

Example 11

1000 hard gelatin capsules, each containing 30 milligrams of m-benzoylhydratropic acid (ketoprofen) as primary active ingredient and 350 milligrams of acetaminophen as potentiating agent are prepared from the following formulation:

| | Grams |
|---|---|
| Ketoprofen | 30 |
| Acetaminophen | 350 |
| Starch | 250 |
| Lactose | 750 |
| Talc | 250 |
| Calcium stearate | 10 |

A uniform mixture of the ingredients is prepared by blending, and used to fill two-piece hard gelatin capsules. The capsules are suitable to be used for providing satisfactory analgesic effect upon administration to subjects suffering from pain.

Example 12

1000 compressed tablets, each containing as the primary active ingredient 20 milligrams of ketoprofen and 425 milligrams of acetaminophen as potentiating agent are prepared from the following formulation:

| | Grams |
|---|---|
| Ketoprofen | 20 |
| Acetaminophen | 425 |
| Starch | 750 |
| Dibasic calcium phosphate hydrous | 5000 |
| Calcium stearate | 2.5 |

The finely powdered ingredients are mixed well and granulated with 10 per cent starch paste. The granulation is dried and compressed into tablets.

Compound F

Example 13

1000 hard gelatin capsules, each containing 85 milligrams of 4-(1,3-dihydro-1-oxo-2H-isoindol-2-yl)-α-methylbenzeneacetic acid (indoprofen) as primary active ingredient and 280 milligrams of acetaminophen as potentiating agent are prepared from the following formulation:

| | Grams |
|---|---|
| Indoprofen | 85 |
| Acetaminophen | 280 |
| Starch | 250 |
| Lactose | 750 |
| Talc | 250 |
| Calcium stearate | 10 |

A uniform mixture of the ingredients is prepared by blending, and used to fill two-piece hard gelatin capsules. The capsules are suitable to be used for providing satisfactory analgesic effect upon administration to subjects suffering from pain.

Example 14

1000 compressed tablets, each containing as the primary active ingredient 35 milligrams of indoprofen and 425 milligrams of acetaminophen as potentiating agent are prepared from the following formulation:

| | Grams |
|---|---|
| Indoprofen | 35 |
| Acetaminophen | 425 |
| Starch | 750 |
| Dibasic calcium phosphate hydrous | 5000 |
| Calcium stearate | 2.5 |

The finely powdered ingredients are mixed well and granulated with 10 per cent starch paste. The granulation is dried and compressed into tablets.

**Claims for the Contracting States: BE CH DE FR GB IT NL SE**

1. A pharmaceutical composition suitable for reducing pain in dosage unit form comprising peripherally active analgesic compound other than acetaminophen and acetaminophen characterised in that it comprises per dosage unit an effective antinociceptive amount in combination of (1) a compound selected from the group consisting of

A) 2-[(2,6-dichlorphenyl)amino]benzeneacetic acid having the formula

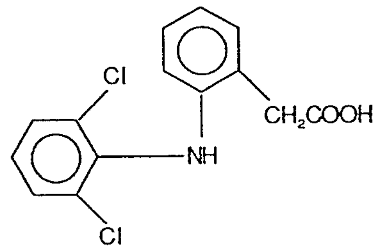

B) (Z)-5-fluoro-2-methyl-1-{[4-(methylsulfinyl)-phenyl]-methylene}1H-indene-3-acetic acid having the formula

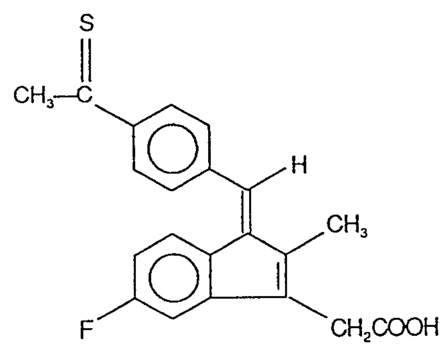

C) 3-(4-biphenylylcarbonyl)propionic acid represented by the formula

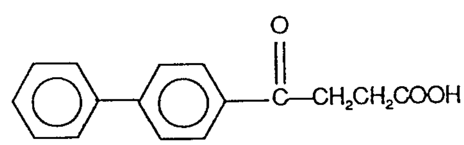

D) 6-methoxy-α-methyl-2-naphthaleneacetic acid having the formula

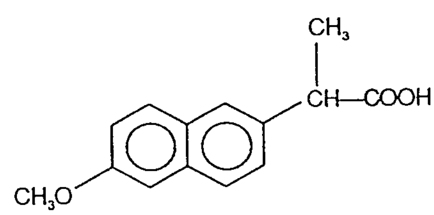

E) m-benzoylhydratropic acid having the formula

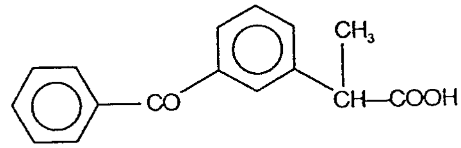

F) 4-(1,3-dihydro-1-oxo-2H-isoindol-2-yl)-α-methylbenzeneacetic acid represented by the formula

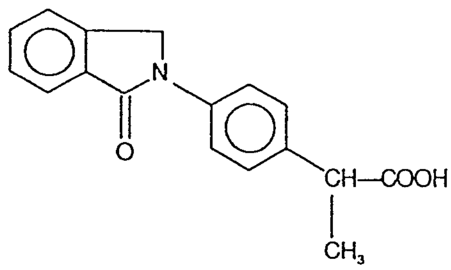

or the non-toxic therapeutically-acceptable salts of any one of said Compounds A) through F) and (2) acetaminophen in a quantity producing a supra-additive effect; and (3) a pharmaceutically-acceptable carrier.

2. A composition according to Claim 1 in which the dosage unit form is a tablet or capsule.

3. A composition according to Claim 1 or Claim 2 which comprises per dosage unit

(1) from 5 to 28 milligrams of 2-[2,6-dichlorophenyl)amino]benzeneacetic acid compound and (2) from 110 to 450 milligrams of acetaminophen.

4. A composition according to Claim 1 or Claim 2 in which the primary antinociceptive agent is sodium 2-[(2,6-dichlorophenyl)amino]benzeneacetate.

5. A composition according to Claim 1 or Claim 2 which comprises per dosage unit (1) from 6 to 100 milligrams of a (Z)-5-fluoro-2-methyl-1-{[4-(methylsulfinyl)phenyl]methylene}-1H-indene-3-acetic acid compound or a salt thereof and (2) from 65 to 450 milligrams of acetaminophen.

6. A compostion according to Claim 1 or Claim 2 in which the primary active agent is (Z)-5-fluoro-2-methyl-1-{[4-(methylsulfinyl)phenyl]methylene}-1H-indene-3-acetic acid.

7. A composition according to Claim 1 or Claim 2 which comprises per dosage unit from 129 to 430 milligrams of the 3-(4-biphenylylcarbonyl)propionic acid compound and from 113 to 450 milligrams of acetaminophen.

8. A composition according to Claim 1 or Claim 2 which comprises per dosage unit from 150 to 400 milligrams of the 3-(4-biphenylylcarbonyl)propionic acid compound and from 125 to 425 milligrams of acetaminophen.

9. A composition according to Claim 1 or Claim 2 which comprises per dosage unit from 85 to 125 milligrams of a 6-methoxy-α-methyl-2-naphthaleneacetic acid compound and from 170 to 450 milligrams of acetaminophen.

10. A composition according to Claim 1 or Claim 2 in which the primary antinociceptive agent is (+)-6-methoxy-α(γ-methyl-2-naphthaleneacetic acid.

11. A composition according to Claim 1 or Claim 2 which comprises per dosage unit from 12 to 50 milligrams of a m-benzoylhydratropic acid compound and from 170 to 450 milligrams of acetaminophen.

12. A composition according to Claim 1 or Claim 2 in which the primary active agent is m-benzoylhydratropic acid.

13. A composition according to Claim 1 or Claim 2 which comprises per dosage unit from 25 to 150 milligrams of the 4-(1,3-dihydro-1-oxo-2H-isoindol-2-yl)-α-methylbenzeneacetic acid compound and from 135 to 450 milligrams of acetaminophen.

14. A composition according to Claim 1 or Claim 2 which comprises per dosage unit from 35 to 130 milligrams of a 4-(1,3-dihydro-1-oxo-2H-isoindol-2-yl)-α-methylbenzeneacetic acid compound and from 150 to 425 milligrams of acetaminophen.

15. A composition in accordance with any one of Claims 1 to 14 for use producing analgesia.

**Claims for the Contracting State: AT**

1. A method of preparing a pharmaceutical composition suitable for reducing pain in dosage unit form comprising intimately admixing an effective antinociceptive amount of

(1) a compound selected from the group consisting of

A) 2-[(2,6-dichlorophenyl)amino]benzeneacetic acid having the formula

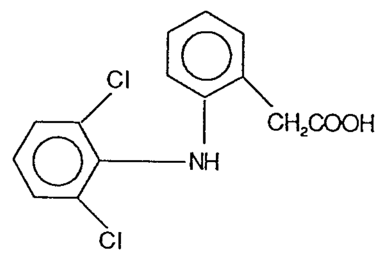

B) (Z)-5-fluoro-2-methyl-1-{[4-(methylsulfinyl)-phenyl]-methylene}1H-indene-3-acetic acid having the formula

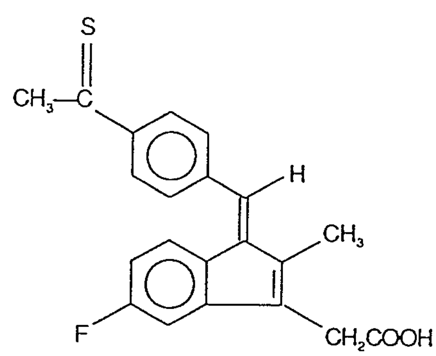

C) 3-(4-biphenylylcarbonyl)propionic acid represented by the formula

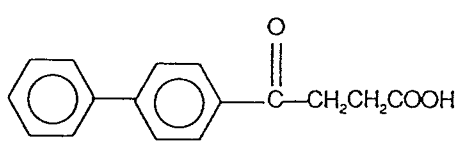

D) 6-methoxy-α-methyl-2-naphthaleneacetic acid having the formula

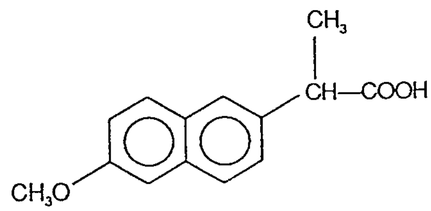

E) m-benzoylhydratropic acid having the formula

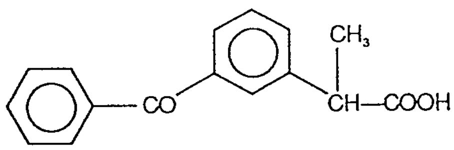

F) 4-(1,3-dihydro-1-oxo-2H-isoindol-2-yl)-α-methylbenzeneacetic acid represented by the formula

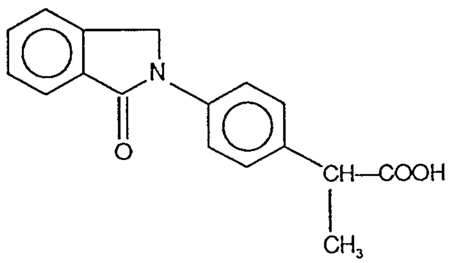

or the non-toxic therapeutically-acceptable salts of any one of said Compounds A) through F) and (2) acetaminophen in a quantity producing a supra-additive effect, and (3) a pharmaceutically-acceptable carrier.

2. A method according to Claim 1 in which the dosage unit form is a tablet or a capsule.

3. A method according to Claim 1 or Claim 2 wherein the dosage unit comprises (1) from 5 to 28 milligrams of 2-[2,6-dichlorophenyl)amino]-benzeneacetic acid compound and (2) from 110 to 450 milligrams of acetaminophen.

4. A method according to Claim 1 or Claim 2 in which the primary antinociceptive agent is sodium 2-[(2,6-dichlorophenyl)amino]benzeneacetate.

5. A method according to Claim 1 or Claim 2 wherein the dosage unit comprises (1) from 6 to 100 milligrams of a (Z)-5-fluoro-2-methyl-1-{[4-(methylsulfinyl)phenyl]methylene}-1H-indene-3-acetic acid compound or a salt thereof and (2) from 65 to 450 milligrams of acetaminophen.

6. A method according to Claim 1 or Claim 2 in which the primary active agent is (Z)-5-fluoro-2-methyl-1-{[4-(methylsulfinyl)phenyl]methylene}-1H-indene-3-acetic acid.

7. A method according to Claim 1 or Claim 2 wherein the dosage unit comprises from 129 to 430 milligrams of the 3-(4-biphenylcarbonyl)propionic acid compound and from 113 to 450 milligrams of acetaminophen.

8. A method according to Claim 1 or Claim 2 wherein the dosage unit comprises from 150 to 400 milligrams of the 3-(4-biphenylcarbonyl)propionic acid compound and from 125 to 425 milligrams of actaminophen.

9. A method according to Claim 1 or 2 wherein the dosage unit comprises from 85 to 125 milligrams of a 6-methoxy-α-methyl-2-naphthaleneacetic acid compound and from 170 to 450 milligrams of acetaminophen.

10. A method according to Claim 1 or Claim 2 in which the primary antinociceptive agent is (+)-6-methoxy-α-methyl-2-naphthaleneacetic acid.

11. A method according to Claim 1 or Claim 2 wherein the dosage unit comprises from 12 to 50 milligrams of a m-benzoylhydratropic acid compound and from 170 to 450 milligrams of acetaminophen.

12. A method according to Claim 1 or Claim 2 in which the primary active agent is m-benzoylhydratropic acid.

13. A method according to Claim 1 or Claim 2 wherein the dosage unit comprises from 25 to 150 milligrams of the 4-(1,3-dihydro-1-oxo-2H-isoindol-2-yl)-α-methylbenzeneactic acid compound and from 135 to 450 milligrams of acetaminophen.

14. A method according to Claim 1 or Claim 2 wherein the dosage unit comprises from 35 to 130 milligrams of a 4-(1,3-dihydro-1-oxo-2H-isoindol-2-yl)α-methylbenzeneacetic acid compound and from 150 to 425 milligrams of acetaminophen.

**Patentansprüche für die Vertragsstaaten: BE CH DE FR GB IT NL SE**

1. Pharmazeutische, zur Linderung von Schmerzen geeignete Zusammensetzung in Dosiseinheitsform, umfassend eine von Acetaminophen unterschiedliche peripher wirksame analgetische Verbindung und Acetaminophen, dadurch gekennzeichnet, dass sie je Dosiseinheit eine antinozizeptiv wirksame Menge in Kombination aus

(1) einer Verbindung, ausgewählt aus der Gruppe bestehend aus

A) 2-[(2,6-Dichlorphenyl)amino]phenylessigsäure mit der Formel

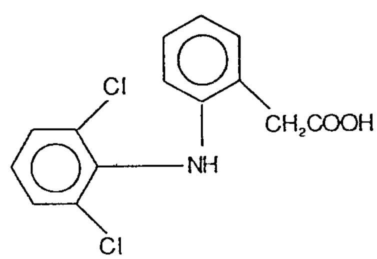

B) (Z)-5-Fluor-2-methyl-1-{[4-(methylsulfinyl)-phenyl]-methylen}-1H-inden-3-essigsäure mit der Formel

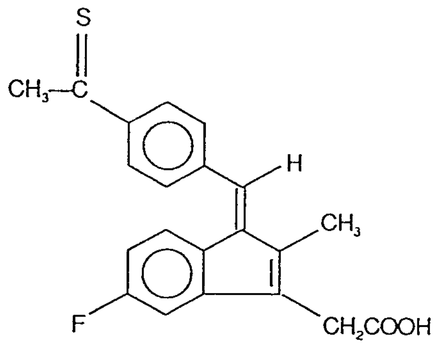

C) 3-(4-Biphenylylcarbonyl)propionsäure entsprechend der Formel

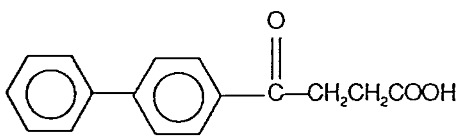

D) 6-Methoxy-α-methyl-2-naphthalin-essigsäure entsprechend der Formel

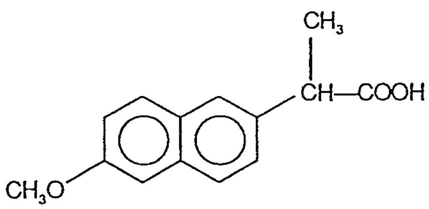

E) m-Benzoylhydratropasäure entsprechend der Formel

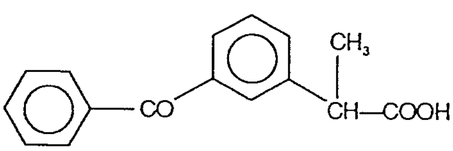

F) 4-(1,3-Dihydro-1-oxo-2H-isoindol-2-yl)-α-methyl-phenylessigsäure entsprechend der Formel

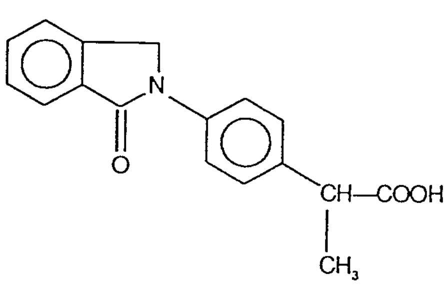

oder den nicht-toxischen therapeutisch annehmbaren Salzen einer der Verbindungen A) bis F); und

(2) Acetaminophen in einer einen supra-additiven Effekt hervorrufenden Menge; und

(3) einen pharmazeutisch annehmbaren Träger umfasst.

2. Zusammensetzung nach Anspruch 1, worin die Dosiseinheitsform eine Tablette oder eine Kapsel ist.

3. Zusammensetzung nach Anspruch 1 oder 2, welche je Dosiseinheit (1) von 5 bis 28 mg 2-[(2,6-Dichlorphenyl) - amino] - phenylessigsäure - Verbindung und (2) von 110 bis 450 mg Acetaminophen enthält.

4. Zusammensetzung nach Anspruch 1 oder 2, worin das primäre antinozizeptive Mittel Natrium-2-[(2,5-dichlorphenyl)-amino]-phenylacetat ist.

5. Zusammensetzung nach Anspruch 1 oder 2, welche je Dosiseinheit (1) von 6 bis 100 mg einer (Z)-5-Fluor-2-methyl-1-{[4-(methylsulfinyl)-phenyl] - methylen} - 1H - inden - 3 - essigsäure-Verbindung oder eines Salzes hievon und (2) von 65 bis 450 mg Acetaminophen enthält.

6. Zusammensetzung nach Anspruch 1 oder 2, worin das primäre aktive Mittel (Z)-5-Fluor-2-methyl-1-{[4-(methylsulfinyl)-phenyl]-methylen}-1H-inden-3-essigsäure ist.

7. Zusammensetzung nach Anspruch 1 oder 2, welche je Dosiseinheit von 129 bis 430 mg der 3 - (4 - Biphenylylcarbonyl)propionsäure -Verbindung und von 113 bis 450 mg Acetaminophen enthält.

8. Zusammensetzung nach Anspruch 1 oder 2, welche je Dosiseinheit von 150 bis 400 mg der 3 - (4 - Biphenylylcarbonyl)propionsäure - Verbindung und von 125 bis 425 mg Acetaminophen enthält.

9. Zusammensetzung nach Anspruch 1 oder 2, welche je Dosisheinheit von 85 bis 125 mg einer 6-Methoxy-α-methyl-2-naphthalin-essigsäure-Verbindung und von 170 bis 450 mg Acetaminophen enthält.

10. Zusammensetzung nach Anspruch 1 oder 2, worin das primäre antinozizeptive Mittel (+)-6-Methoxy-α-methyl-2-naphthalin-essigsäure ist.

11. Zusammensetzung nach Anspruch 1 oder 2, welche je Dosiseinheit von 12 bis 50 mg einer m-Benzoylhydratropasäure-Verbindung und von 170 bis 450 mg Acetaminophen enthält.

12. Zusammensetzung nach Anspruch 1 oder 2, worin das primäre aktive Mittel m-Benzoylhydratropasäure ist.

13. Zusammensetzung nach Anspruch 1 oder 2, welche je Dosiseinheit von 25 bis 150 mg der 4-(1,3-Dihydro-1-oxo-2H-isoindol-2-yl)-α-methylphenylessigsäure-Verbindung und von 135 bis 450 mg Acetaminophen enthält.

14. Zusammensetzung nach Anspruch 1 oder 2, welche je Dosiseinheit von 35 bis 130 mg einer 4-(1,3-Dihydro-1-oxo-2H-isoindol-2-yl)-α-methylphenylessigsäure-Verbindung und von 150 bis 425 mg Acetaminophen enthält.

15. Zusammensetzung nach einem der Ansprüche 1 bis 14 zur Anwendung bei der Bewirkung von Schmerzlinderung.

## Patentansprüche für den Vertragsstaat: AT

1. Verfahren zur Herstellung einer pharmazeutischen, zur Linderung von Schmerzen geeigneten Zusammensetzung in Form von Dosiseinheiten, umfassend das innige Vermischen einer antinozizeptiv wirksamen Menge von (1) einer Verbindung ausgewählt aus der Gruppe bestehend aus

A) 2-[(2,6-Dichlorphenyl)amino]phenylessigsäure mit der Formel

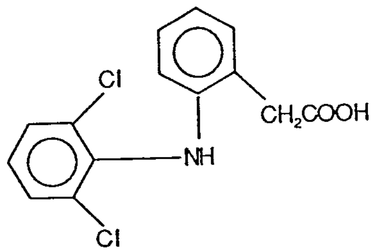

B) (Z)-5-Fluor-2-methyl-1-{[4-(methylsulfinyl)-phenyl]methylen}-1H-inden-3-essigsäure mit der Formel

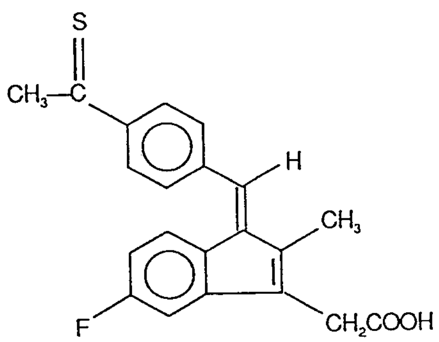

C) 3-(4-Biphenylylcarbonyl)propionsäure entsprechend der Formel

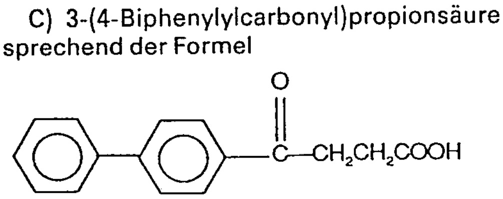

D) 6-Methoxy-α-methyl-2-naphthalin-essigsäure entsprechend der Formel

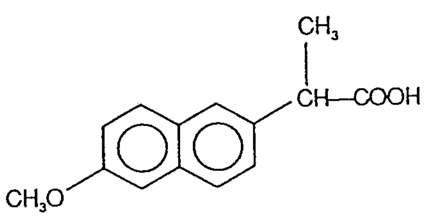

E) m-Benzoylhydratropasäure entsprechend der Formel

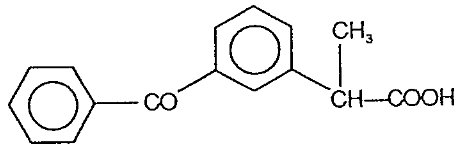

F) 4-(1,3-Dihydro-1-oxo-2H-isoindol-2-yl)-α-methylphenylessigsäure entsprechend der Formel

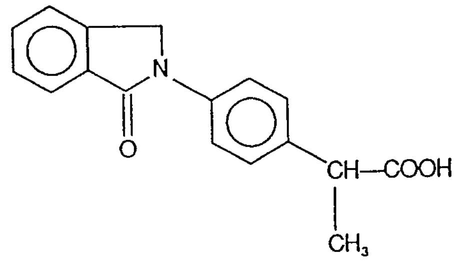

oder den nicht-toxischen therapeutisch annehmbaren Salzen einer der Verbindungen A) bis F); und

(2) Acetaminophen in einer supra-additiven Effekt hervorrufenden Menge, und

(3) einem pharmazeutisch annehmbaren Träger.

2. Verfahren nach Anspruch 1, worin die Dosiseinheitsform eine Tablette oder eine Kapsel ist.

3. Verfahren nach Anspruch 1 oder 2, worin die Dosiseinheit

(1) von 5 bis 28 mg 2-[(2,6-Dichlorphenyl)-amino]-phenylessigsäure-Verbindung und

(2) von 110 bis 450 mg Acetaminophen enthält.

4. Verfahren nach Anspruch 1 oder 2, worin das primäre antinozizeptive Mittel Natrium-2-[(2,5-dichlorphenyl)-amino]-phenylacetat ist.

5. Verfahren nach Anspruch 1 oder 2, worin die Dosiseinheit

(1) von 6 bis 100 mg einer (Z)-5-Fluor-2-methyl-1-{[4-(methylsulfinyl)-phenyl]-methylen{-1H-inden-3-essigsäure-Verbindung oder eines Salzes hievon und

(2) von 65 bis 450 mg Acetaminophen enthält.

6. Verfahren nach Anspruch 1 oder 2, worin das primäre aktive Mittel (Z)-5-Fluor-2-methyl-1-{[4-(methylsulfinyl)-phenyl]-methylen{-1H-inden-3-essigsäure ist.

7. Verfahren nach Anspruch 1 oder 2, worin die Dosiseinheit von 129 bis 430 mg der 3-(4-Bisphenylylcarbonyl)-propionsäure-Verbindung und von 113 bis 450 mg Acetaminophen enthält.

8. Verfahren nach Anspruch 1 oder 2, worin die Dosiseinheit von 150 bis 400 mg der 3-(4-Biphenylylcarbonyl)-propionsäure-Verbindung und von 125 bis 425 mg Acetaminophen enthält.

9. Verfahren nach Anspruch 1 oder 2, worin die Dosiseinheit von 85 bis 125 mg einer 6-Methoxy-α-methyl-2-naphthalin-essigsäure-Verbindung und von 170 bis 450 mg Acetaminophen enthält.

10. Verfahren nach Anspruch 1 oder 2, worin das primäre antinozizeptive Mittel (+)-6-Methoxy-α-methyl-2-naphthalinessigsäure ist.

11. Verfahren nach Anspruch 1 oder 2, worin die Dosiseinheit von 12 bis 50 mg einer m-Benzoylhydratropasäure-Verbindung und von 170 bis 450 mg Acetaminophen enthält.

12. Verfahren nach Anspruch 1 oder 2, worin das primäre aktive Mittel m-Benzoylhydratropasäure ist.

13. Verfahren nach Anspruch 1 oder 2, worin

die Dosiseinheit von 25 bis 150 mg der 4 - (1,3 - Dihydro - 1 - oxo - 2H - isoindol - 2 - yl) - α - methyl-phenylessigsäure-Verbindung und von 135 bis 450 mg Acetaminophen enthält.

14. Verfahren nach Anspruch 1 oder 2, worin die Dosiseinheit von 35 bis 130 mg einer 4-(1,3-Dihydro-1-oxo-2H-isoindol-2-yl)-α-methyl-phenylessigsäure-Verbindung und von 150 bis 425 mg Acetaminophen enthält.

**Revendications pour les états contractants: BE CH DE FR GB IT NL SE**

1. Une composition pharmaceutique convenant pour réduire la douleur en une forme unitaire d'administration comprenant un composé analgésique actif périphériquement autre que l'acétaminophène et l'acétaminophène, caractérisé en ce qu'il comprend par dose unitaire une quantité antinociceptife efficace en combinaison de:

(1) un composé choisi dans le groupe constitué par:

(A) l'acide 2-[(2,6-dichlorophényl)amino]benzène-acétique de formule:

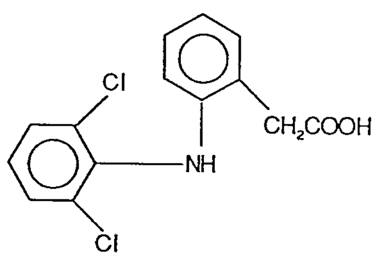

B) l'acide(Z)-5-fluoro-2-méthyl-1-{[4-(méthylsulfinyl)phényl]méthylène}-1H-indène-3-acétique de formule:

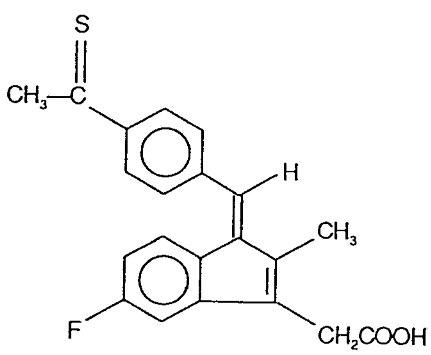

C) l'acide 3-(4-biphénylylcarbonyl)propionique représenté par la formule:

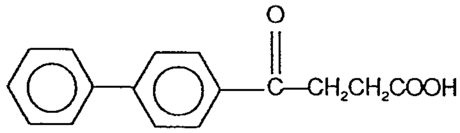

D) l'acide 6-méthoxy-α-méthyl-2-naphthalène-acétique de formule:

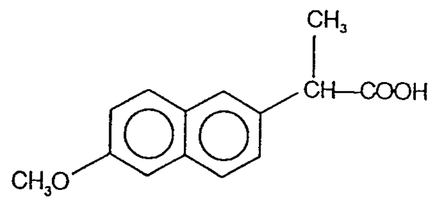

E) l'acide m-benzoylhydratropique de formule:

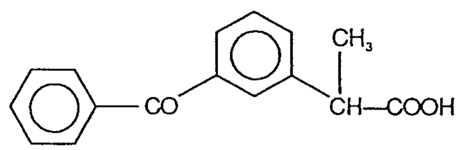

F) l'acide 4-(1,3-dihydro-1-oxo-2H-isoindole-2-yl)-α-méthylbenzène-acétique représenté par la formule:

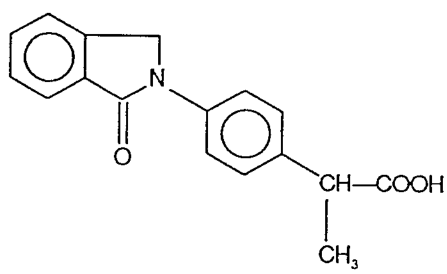

ou les sels non toxiques acceptables en thérapeutique de l'un quelconque desdits composés A) à F); et

(2) l'acétaminophène en quantité produisant des effects supra additifs; et

(3) un véhicule acceptable en pharmacie.

2. Une composition selon la revendication 1, dans laquelle la forme unitaire d'administration est un comprimé ou une capsule.

3. Une composition selon la revendication 1 ou la revendication 2, qui comprend par dose unitaire (1) 5 à 28 milligrammes d'un composé de type 2-[(2,6-dichlorophényl)amino]benzène-acétique et (2) 110 à 450 milligrammes d'acétaminophène.

4. Une composition selon la revendication 1 ou la revendication 2, dans laquelle l'agent antinociceptif principal est le 2-[(2,6-dichlorophényl)amino]-benzène-acétate de sodium.

5. Une composition selon la revendication 1 ou la revendication 2 qui comprend par dose unitaire (1) de 6 à 100 milligrammes d'acide (Z)-5-fluoro-2-méthyl-1-{[4-(méthylsulfinyl)phényl]méthylène}-1H-indène-3-acétique ou un sel correspondant et (2) de 65 à 450 milligrammes d'acétaminophène.

6. Une composition selon la revendication 1 ou la revendication 2, dans laquelle l'agent actif principal est l'acide (Z)-5-fluoro-2-méthyl-1-{[4-(méthylsulfinyl)phényl]méthylène}-1H-indène-3-acétique.

7. Une composition selon la revendication 1 ou la revendication 2 qui comprend par dose unitaire, de 129 à 430 mg de composé de type acide 3-(4-biphénylylcarbonyl)propionique et de 113 à 450 mg d'acétaminophène.

8. Une composition selon la revendication 1 ou la revendication 2, qui comprend par dose unitaire de 150 à 400 mg du composé de type acide 3-(4-biphénylcarbonyl)propionique et de 125 à 425 mg d'acétaminophène.

9. Une composition selon la revendication 1 ou la revendication 2 qui comprend par dose unitaire de 85 à 125 milligrammes de composé de type acide 6-méthoxy-α-méthyl-2-naphtalène-acétique et de 170 à 450 milligrammes d'acétaminophène.

10. Une composition selon la revendication 1 ou la revendication 2, dans laquelle l'agent antinociceptif principal est l'acide (+)-6-méthoxy-α-méthyl-2-naphtalène-acétique.

11. Une composition selon la revendication 1 ou la revendication 2 qui comprend par dose unitaire de 12 à 50 mg d'un composé de type acide m-benzoyl-hydratropique et de 170 à 450 mg d'acétaminophène.

12. Une composition selon la revendication 1 ou la revendication 2 dans laquelle l'agent actif principal est l'acide m-benzoylhydratropique.

13. Une composition selon la revendication 1 ou la revendication 2 qui comprend par dose unitaire de 25 à 150 milligrammes du composé de type acide 4-(1,3-dihydro-1-oxo-2H-isoindole-2-yl)-α-méthylbenzène-actique et de 135 à 450 milligrammes d'acétaminophène.

14. Une composition selon la revendication 1 ou la revendication 2 qui comprend par dose unitaire de 35 à 130 milligrammes d'un composé de type 4-(1,3-dihydro-1-oxo-2H-isoindole-2-yl)-α-méthylbenzène-acétique et de 150 à 425 milligrammes d'acétaminophène.

15. Une composition selon l'une quelconque des revendications 1 à 14 pour l'emploi pour produire une analgésie.

**Revendications pour l'état contractant: AT**

1. Un procédé pour préparer une composition pharmaceutique convenant pour réduire la douleur sous forme d'une dose unitaire d'administration, comprenant le mélange intime d'une quantité antinociceptive efficace de

(1) un composé choisi parmi le groupe constitué par A) l'acide 2-[(2,6-dichlorophényl)amino]benzène-acétique de formule:

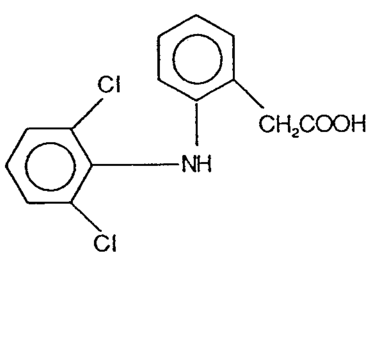

B) l'acide (Z)-5-fluoro-2-méthyl-1-{[4-(méthylsulfinyl)phényl]méthylène}-1H-indène-3-acétique de formule:

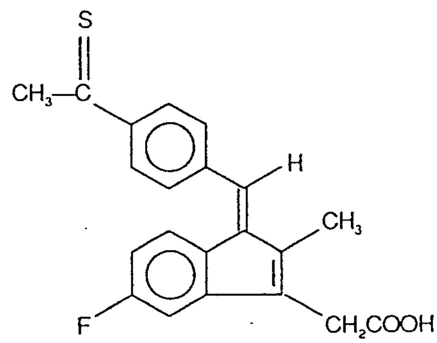

C) l'acide 3-(4-biphénylylcarbonyl)propionique représenté par la formule:

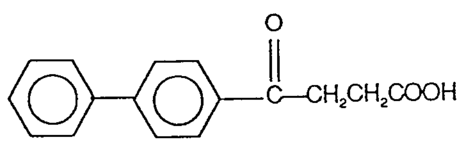

D) l'acide 6-méthoxy-α-méthyl-2-naphtalène-acétique de formule:

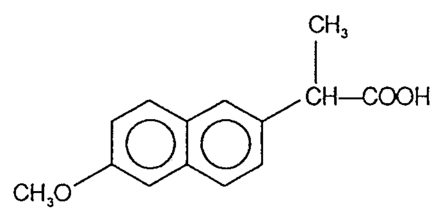

E) l'acide m-benzoylhydratropique de formule:

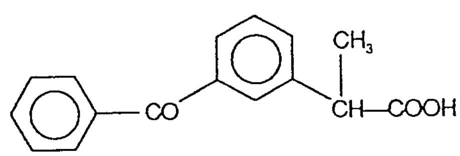

F) l'acide 4-(1,3-dihydro-1-oxo-2H-isoindole-2-yl)-α-méthylbenzène-acétique représenté par la formule:

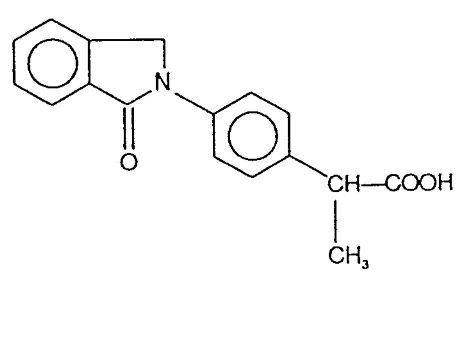

ou les sels non toxiques acceptables en thérapeutique de l'un quelconque desdits composés A) à F); et

(2) l'acétaminophène en quantité produisant des effets supra-additifs; et

(3) un véhicule acceptable en pharmacie.

2. Un procédé selon la revendication 1 dans lequel la forme unitaire d'administration est un comprimé ou une capsule.

3. Un procédé selon la revendication 1 ou la revendication 2 dans lequel la dose unitaire comprend (1) de 5 à 28 milligrammes d'un composé de type acide 2-[(2,6-dichlorophényl)amino]benzène-acétique et (2) de 110 à 450 milligrammes d'acétaminophène.

4. Un procédé selon la revendication 1 ou la revendication 2, dans lequel l'agent antinociceptif principal est le 2-[(2,6-dichlorophényl)amino]benzène-acétate de sodium.

5. Un procédé selon la revendication 1 ou la revendication 2 où la dose unitaire comprend (1) de 6 à 100 milligrammes d'un composé de type acide (Z)-5-fluoro-2-méthyl-1-{[4-(méthylsulfinyl)phényl]méthylène}-1H-indène-3-acétique ou un sel correspondant et (2) de 65 à 450 milligrammes d'acétaminophène.

6. Un procédé selon la revendication 1 ou la revendication 2 dans lequel l'agent actif principal est l'acide (Z)-5-fluoro-2-méthyl-1-{[4-(méthylsulfinyl)-phényl]méthylène}-1H-indène-3-acétique.

7. Un procédé selon la revendication 1 ou la revendication 2, dans lequel la dose unitaire comprend de 129 à 430 milligrammes du composé de type acide 3-(4-biphénylylcarbonyl)propionique et de 113 à 450 milligrammes d'acétaminophène.

8. Un procédé selon la revendication 1 ou la revendication 2 dans lequel la dose unitaire comprend de 150 à 400 milligrammes du composé de type acide 3-(4-biphénylylcarbonyl)propionique et de 125 à 425 milligrammes d'acétaminophène.

9. Un procédé selon la revendication 1 ou la revendication 2 dans lequel la dose unitaire comprend de 85 à 125 milligrammes d'un composé de type acide 6-méthoxy-α-méthyl-2-naphtalène-acétique et de 170 à 450 milligrammes d'acétaminophène.

10. Un procédé selon la revendication 1 ou la revendication 2 dans lequel l'agent antinociceptif principal est l'acide (+)-6-méthoxy-α-méthyl-2-naphtalène-acétique.

11. Un procédé selon la revendication 1 ou la revendication 2 dans lequel la dose unitaire comprend de 12 à 50 milligrammes d'un composé de type acide m-benzoylhydratropique et de 170 à 450 milligrammes d'acétaminophène.

12. Un procédé selon la revendication 1 ou la revendication 2 dans lequel l'agent actif principal est l'acide m-benzoylhydratropique.

13. Un procédé selon la revendication 1 ou la revendication 2 dans lequel la dose unitaire comprend de 25 à 150 milligrammes du composé de type acide 4-(1,3-dihydro-1-oxo-2H-isoindole-2-yl)-α-méthylbenzène-acétique et de 135 à 450 milligrammes d'acétaminophène.

14. Un procédé selon la revendication 1 ou la revendication 2 dans lequel la dose unitaire comprend de 35 à 130 milligrammes d'un composé de type acide 4-(1,3-dihydro-1-oxo-2H-isoindole-2-yl)-α-méthylbenzène-acétique et de 150 à 425 milligrammes d'acétaminophène.